(19)

Europäisches
Patentamt
European
Patent Office
Office européen
des brevets

(11)  **EP 1 801 589 A1**

(12)  **EUROPEAN PATENT APPLICATION**
published in accordance with Art. 158(3) EPC

(43) Date of publication:
**27.06.2007 Bulletin 2007/26**

(51) Int Cl.:
*G01N 33/543* (2006.01)    *G01N 33/553* (2006.01)
*G01N 33/566* (2006.01)

(21) Application number: **05774524.2**

(22) Date of filing: **29.08.2005**

(86) International application number:
**PCT/JP2005/015686**

(87) International publication number:
**WO 2006/022411 (02.03.2006 Gazette 2006/09)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **27.08.2004 JP 2004248993**

(71) Applicants:
• **National Institute of Advanced Industrial Science and Technology**
  **Tokyo 100-8921 (JP)**
• **PRECISION SYSTEM SCIENCE CO., LTD.**
  **Matsudo-shi,**
  **Chiba, 271-0064 (JP)**

(72) Inventors:
• **MACHIDA, Masayuki,**
  **National Inst of Adv Ind S & T**
  **Tsukuba-shi,**
  **Ibaraki 305-8566 (JP)**

• **NATSUKI, Jun,**
  **National Inst of Adv Indust. S & T**
  **Tsukuba-shi,**
  **Ibaraki 305-8566 (JP)**
• **TAMAMO, Koichi,**
  **National Inst of Adv. Indst. S & T**
  **Tsukuba-shi,**
  **Ibaraki 305-8566 (JP)**
• **TAJIMA, Hideji,**
  **Precision System Science Co., Ltd**
  **Matsudo-shi, Chiba 271-0064 (JP)**

(74) Representative: **Woods, Geoffrey Corlett et al**
  **J.A. KEMP & CO.**
  **Gray's Inn**
  **14 South Square**
  **London WC1R 5JJ (GB)**

(54)  **METHOD OF DETECTING STRUCTURAL CHANGE IN TARGET SUGAR CHAIN**

(57)    A method of easily and efficiently detecting any structural change in a target sugar chain that can be present as a first or a second sugar chain depending on the structural change. In the method, the target sugar chain is mixed with a first to an nth lectin that can bind to the first and second sugar chains and the ratio between the amounts of the first and second lectins as bound to the target sugar chain is used as an index for detecting the presence or absence of a structural change in the target sugar chain.

EP 1 801 589 A1

**Description**

TECHNICAL FIELD

The present invention relates to a method for detecting structural changes in a target sugar chain.

BACKGROUND ART

A variety of causes can change the structure of a sugar chain. For example, sugar chains contained in $\alpha$-fetoprotein (AFP) may have an $\alpha$-L-fucose residue, N-acetyl glucosamine residue (bisecting N-acetyl glucosamine), and the like added in association with the advance of hepatocellular cancer. Consequently, it appears that hepatocellular cancer can be diagnosed by detecting the structural changes of the sugar chain contained in the AFP.

To detect the structural changes in a target sugar which can be present as a first or second sugar chain depending on structural change, the target sugar chain may be mixed with a lectin that binds to the first sugar chain by a different degree than to the second sugar chain and the degree of lectin binding to the target sugar chain can be used as an index (International Publication WO2002/066634 pamphlet). Nonetheless, in this case, it is necessary to investigate the behavior of binding between the target sugar chain and the lectin in detail and obtain information relating to the binding properties, such as binding constant.

In the case of a glycoprotein, where the first sugar chain and second sugar chain have different sugar chain structures but the protein structure is substantially the same, in order to detect the structural change in the target sugar chain, the target sugar chain, a lectin that recognizes a certain sugar chain structure that has the first sugar chain but does not have the second sugar chain, and an antibody that will bind to the first and second sugar chains to which the lectin has not been bound but which will be prevented from binding to the first sugar chain to which the lectin has been bound may be mixed together, it is determined as an index whether or not the antibody binds to the target sugar chain (Japanese Patent No. 3070418). Nonetheless, in this case, it is necessary to use a special antibody that will bind to the first and second sugar chains to which lectin has not been bound but which will be prevented from binding to the first sugar chain to which the lectin has been bound.

DISCLOSURE OF THE INVENTION

An object of the present invention is to provide a method that can simply and efficiently detect structural changes in target sugar chains which can be present as a first or second sugar chain depending on structural change.

In order to achieve the aforementioned object, the present invention provides a method for detecting the presence or absence of a structural change in a target sugar chain, in which the target sugar chain which can be present as a first or second sugar chain depending on the structural change is mixed with a first to an $n^{th}$ lectin (n is an integer of 2 or more) that can bind to the first and second sugar chains, and the ratio between the amount of the first lectin binding to the sugar chain and the amount of the second lectin binding to the sugar chain is used as an index, the first to the $n^{th}$ lectin satisfying the following relation (I):

Relation 1

$$\frac{B_{11}/B_{12}}{B_{21}/B_{22}} > \frac{A_{11}/A_{12}}{A_{21}/A_{22}} > 1$$

[wherein, $A_{11}$ represents the amount of the first lectin binding to one molecule of the first sugar chain when the first sugar chain and the first lectin are mixed in the absence of another lectin; $A_{12}$ represents the amount of the second lectin binding to one molecule of the first sugar chain when the first sugar chain and the second lectin are mixed in the absence of another lectin; $A_{21}$ represents the amount of the first lectin binding to one molecule of the second sugar chain when the second sugar chain and the first lectin are mixed in the absence of another lectin; $A_{22}$ represents the amount of the second lectin binding to one molecule of the second sugar chain when the second sugar chain and the second lectin are mixed in the absence of another lectin; $B_{11}$ represents the amount of the first lectin binding to one molecule of the first sugar chain when the first sugar chain is mixed with the first to the $n^{th}$ lectin; $B_{12}$ represents the amount of the second lectin binding to one molecule of the first sugar chain when the first sugar chain is mixed with the first to the $n^{th}$ lectin; $B_{21}$ represents the amount of the first lectin binding to one molecule of the second sugar chain when the second sugar chain is mixed with the first to the $n^{th}$ lectin; and $B_{22}$ represents the amount of the second lectin binding to one molecule of the second sugar chain when the second sugar chain is mixed with the first to the $n^{th}$ lectin.]

There are three possibilities for the occurrence of the target sugar chain: it may be present only as the first sugar chain, only as the second sugar chain, or as a mixture of the first and second sugar chains. Specifically, the total number of molecules (N) of the target sugar chain may be represented as [the number $(N_1)$ of molecules of the target sugar chain present as the first sugar chain] + [the number $(N_2)$ of molecules of the target sugar chain present as the second sugar chain]. Note that $N_1$ and $N_2$ each represent an integer of 0 or more, but that $N_1$ and $N_2$ are not both 0 at the same time.

The amount of the first lectin binding to the target sugar chain $(D_1)$ when the target sugar chain has been mixed with the first to the $n^{th}$ lectin is represented by [the amount of the first lectin binding to one molecule of the first sugar chain $(B_{11})$] $\times$ [the number $(N_1)$ of molecules of the target sugar chain present as the first sugar chain] + [the amount of the first lectin binding to one molecule of the second sugar chain $(B_{21})$] $\times$ [the number $(N_2)$ of molecules of the target sugar chain present as the second sugar chain], and the amount of the second lectin binding to the target sugar chain $(D_2)$ when the target sugar chain has been mixed with the first to the $n^{th}$ lectin is represented by [the amount of the second lectin binding to one molecule of the first sugar chain $(B_{12})$] $\times$ [the number $(N_1)$ of molecules of the target sugar chain present as the first sugar chain] + [the amount of the second lectin binding to one molecule of the second sugar chain $(B_{22})$] $\times$ [the number $(N_2)$ of molecules of the target sugar chain present as the second sugar chain].

When the first to the $n^{th}$ lectin that satisfy the aforementioned relation (I) are used, the ratio $(D_1/D_2$ or $D_2/D_1)$ between the amount of the first lectin binding to the target sugar chain $(D_1)$ when the target sugar chain has been mixed with the first to the $n^{th}$ lectin and the amount of the second lectin binding to the target sugar chain $(D_2)$ when the target sugar chain has been mixed with the first to the $n^{th}$ lectin varies in the following manner depending on the state in which the target sugar chain is present (specifically, on the values of $N_1$ and $N_2$).

(1) If the target sugar chain is present only as the first sugar chain $(N_1 \geq 1, N_2 = 0)$

$$D_1 / D_2 = B_{11} / B_{12}$$

$$D_2 / D_1 = B_{12} / B_{11}$$

(2) If the target sugar chain is present only as the second sugar chain $(N_i = 0, N_2 \geq 1)$

$$D_1 / D_2 = B_{21} / B_{22}$$

$$D_2 / D_1 = B_{22} / B_{21}$$

(3) If the target sugar chain is present as a mixture of the first and second sugar chains $(N_1 \geq 1, N_2 \geq 1)$

$$B_{11} / B_{12} \ > \ D_1 / D_2 \ > \ B_{21} / B_{22}$$

$$B_{12} / B_{11} \ < \ D_2 / D_1 \ < \ B_{22} / B_{21}$$

Note also that "$B_{11}/B_{12}$", "$B_{12}/B_{11}$" , "$B_{21}/B_{22}$", "$B_{22}/B_{21}$", "$D_1/D_2$" and "$D_2/D_1$" mean not only fixed values but also mean fixed value ranges. In addition, "=" means not only complete identicalness, but also being substantially the

EP 1 801 589 A1

same.

Consequently, the ratio ($D_1/D_2$ or $D_2/D_1$) between the amount of the first lectin binding to the target sugar chain ($D_1$) when the target sugar chain has been mixed with the first to the $n^{th}$ lectin and the amount of the second lectin binding to the target sugar chain ($D_2$) when the target sugar chain has been mixed with the first to the $n^{th}$ lectin can be used as an index for detecting the presence or absence of a structural change in the target sugar chain. Specifically, in the case where the first sugar chain is the sugar chain prior to the structural change and the second sugar chain is the sugar chain after the structural change, if $B_{11}/B_{12} > D_1/D_2 \geq B_{21}/B_{22}$ or $B_{12}/B_{11} < D_2/D_1 \leq B_{22}/B_{21}$, then it can be determined that the target sugar chain is present only as the second sugar chain or as a mixture of the first and second sugar chains, in other words, the structural change has occurred in the target sugar chain. Moreover, in the case where the second sugar chain is the sugar chain prior to the structural change and the first sugar chain is the sugar chain after the structural change, if $B_{11}/B_{12} \geq D_1/D_2 > B_{21}/B_{22}$ or $B_{12}/B_{11} \leq D_2/D_1 < B_{22}/B_{21}$, then it can be determined that the target sugar chain is present only as the first sugar chain or as a mixture of the first and second sugar chains, in other words, the structural change has occurred in the target sugar chain.

In addition, the ratio ($C_1/C_2$ or $C_2/C_1$) between the amount of the first lectin binding to the target sugar chain ($C_1$) when the target sugar chain and the first lectin have been mixed in the absence of another lectin and the amount of the second lectin binding to the target sugar chain ($C_2$) when the target sugar chain and the second lectin have been mixed in the absence of another lectin can also be used as an index for detecting the presence or absence of a structural change in the target sugar chain in the same way as the ratio $D_1/D_2$ or $D_2/D_1$.

Nonetheless, when using $C_1/C_2$ or $C_2/C_1$ as an index, $C_1$ is measured under the condition of mixing the target sugar chain and the first lectin in the absence of another lectin, and $C_2$ is measured under the condition of mixing the target sugar chain and the second lectin in the absence of another lectin, and therefore $C_1$ and $C_2$ must be measured in separate reaction systems. Moreover, the states in which the target sugar chain is present in the respective reaction systems (specifically, the values of $N_1$ and $N_2$) must be identical.

In contrast, when using $D_1/D_2$ or $D_2/D_1$ as an index, $D_1$ and $D_2$ are measured under the same conditions of mixing the target sugar chain with the first to the $n^{th}$ lectin, and therefore, they can be measured in the same reaction system. If measurements are conducted in the same reaction system, the states in which the target sugar chain is present during $D_1$ and $D_2$ measurements (specifically, the values of $N_1$ and $N_2$) are identical.

Consequently, the presence or absence of structural changes in the target sugar chain can be detected more easily and efficiently by using $D_1/D_2$ or $D_2/D_1$ as the index rather than using $C_1/C_2$ or $C_2/C_1$ as the index.

Moreover, the fluctuations of measured values obtained when $D_1$ and $D_2$ are measured a plurality of times in the same reaction system will be smaller than the fluctuations of measured values obtained when $C_1$ and $C_2$ are measured a plurality of times in different reaction systems.

Consequently, the presence or absence of structural changes in the target sugar chain can be detected more precisely by using $D_1/D_2$ or $D_2/D_1$ as the index rather than using $C_1/C_2$ or $C_2/C_1$ as the index.

Further, by using the first to the $n^{th}$ lectin that satisfy the aforementioned relation (I), $B_{11}/B_{12}$ and $B_{21}/B_{22}$ can be distinguished more clearly than are $A_{11}/A_{12}$ and $A_{21}/A_{22}$, and, in addition, $B_{12}/B_{11}$ and $B_{22}/B_{21}$ can be distinguished more clearly than are $A_{12}/A_{11}$ and $A_{22}/A_{21}$.

Consequently, the presence or absence of structural changes in the target sugar chain can be detected more precisely by using $D_1/D_2$ or $D_2/D_1$ as the index rather than using $C_1/C_2$ or $C_2/C_1$ as the index.

Because the first and second sugar chains have a structural difference in sugar chain as produced by the structural change, $A_{11}$ can indicate a value or value range different from that of $A_{21}$. Similarly, $A_{12}$ can indicate a value or value range different from that of $A_{22}$. Consequently, $A_{11}/A_{12}$ can indicate a value or value range different from that of $A_{21}/A_{22}$. In the method of the present invention, a first and a second lectin that satisfy $A_{11}/A_{12} > A_{21}/A_{22}$ are selected.

$A_{11}$ is such that when the first sugar chain has been mixed with the first to the $n^{th}$ lectin, it is substantially the same as the amount of the first lectin binding to one molecule of the first sugar chain in the case where the binding of the first lectin to the first sugar chain is not affected by the binding of another lectin to the first sugar chain. Consequently, in the case where the first sugar chain has been mixed with the first to the $n^{th}$ lectin, $B_{11}$ can indicate a value or value range different than $A_{11}$ if the binding of the first lectin to the first sugar chain is affected by the binding of another lectin to the first sugar chain. Similarly, in the case where the first sugar chain has been mixed with the first to the $n^{th}$ lectin, $B_{12}$ can indicate a value or value range different than $A_{12}$ if the binding of the second lectin to the first sugar chain is affected by the binding of another lectin to the first sugar chain. Consequently, in the case where the first sugar chain has been mixed with the first to the $n^{th}$ lectin, $B_{11}/B_{12}$ can indicate a value or value range different than $A_{11}/A_{12}$ if the bindings of the first and second lectins to the first sugar chain are affected respectively by the binding of another lectin to the first sugar chain. Similarly, in the case where the second sugar chain has been mixed with the first to the $n^{th}$ lectin, $B_{21}/B_{22}$ can indicate a value or value range different than $A_{21}/A_{22}$ if the bindings of the first and second lectins to the second sugar chain are affected respectively by the binding of another lectin to the second sugar chain. In the method of the present invention, the first to the $n^{th}$ lectin that satisfy the aforementioned relation (I) are selected.

Further, any of the following relations (i) to (iv) may be cited as exemplary cases where the aforementioned relation (I) is established.

$$B_{11}/B_{12} > A_{11}/A_{12} > A_{21}/A_{22} \geq B_{21}/B_{22} \quad (\text{i})$$

$$B_{11}/B_{12} \geq A_{11}/A_{12} > A_{21}/A_{22} > B_{21}/B_{22} \quad (\text{ii})$$

$$A_{11}/A_{12} \geq B_{11}/B_{12} > A_{21}/A_{22} > B_{21}/B_{22} \quad (\text{iii})$$

$$B_{11}/B_{12} > A_{11}/A_{12} > B_{21}/B_{22} \geq A_{21}/A_{22} \quad (\text{iv})$$

In the method of the present invention, as long as the first to $n^{th}$ lectins can bind to the first and second sugar chains such that they satisfy the aforementioned relation (I), the first to $n^{th}$ lectins are not particularly limited. Preferably, the first to $n^{th}$ lectins can respectively bind to specified sugar chain structures in a specific manner. In that case, the behavior of binding of the first to $n^{th}$ lectins to the first and second sugar chains can be easily ascertained, so it is easy to select the first to $n^{th}$ lectins that satisfy the aforementioned relation (I). Here, the specific affinity of lectins to specified sugar chain structures is not generally a strict one and the lectins can also usually show affinity to sugar chain structures other than the specified sugar chain structures.

In the method of the present invention, if n is small, then the behavior of binding of the first to $n^{th}$ lectins to the first and second sugar chains is easily ascertained, so it is easy to select the first to $n^{th}$ lectins that satisfy the aforementioned relation (I). As long as n is an integer of 2 or more, n is not particularly limited, but preferably, it is 2 to 5, and more preferably, 2 to 3.

In the method of the present invention, if the difference in sugar chain structure between the first and second sugar chains is slight, the difference between the behavior of binding of the first to $n^{th}$ lectins to the first sugar chain and the behavior of binding of the first to $n^{th}$ lectins to the second sugar chains is easily ascertained, so it is easy to select the first to $n^{th}$ lectins that satisfy the aforementioned relation (I).

The case where the difference in sugar chain structure between the first and second sugar chains is slight may be exemplified by the case where the structures of the two sugar chains differ in that the first sugar chain has a first sugar chain structure to which a first lectin can bind and the second sugar chain does not have that first sugar chain structure and the two sugar chain structures are substantially the same on all other points. In that case, $A_{11}$ is larger than $A_{21}$ while $A_{12}$ and $A_{22}$ are substantially the same, and therefore $A_{11}/A_{12} > A_{21}/A_{22}$ is established. Further, the expression reading "the two sugar chain structures are substantially the same" may also cover the case where those parts of the first and second sugar chains to which the first to $n^{th}$ lectins cannot bind have differences in sugar chain structure.

In the case where the structures of the two sugar chains differ in that the first sugar chain has a first sugar chain structure to which a first lectin can bind and the second sugar chain does not have that first sugar chain structure and the two sugar chain structures are substantially the same on all other points, $B_{11}/B_{12} > A_{11}/A_{12}$ is established by selecting the first to $n^{th}$ lectins such that the second lectin is suppressed from binding to the first sugar chain if the first lectin binds to the first sugar chain structure of the first sugar chain. Note that in the case where the second lectin can bind to a plurality of sugar chain structures in the first sugar chain, the second lectin may be suppressed from binding to one or more of these sugar chain structures.

Suppose $B_{11}/B_{12} > A_{11}/A_{12}$ is established. When the first to $n^{th}$ lectins have been mixed with the second sugar chain and if the bindings of the first and second lectins to the second sugar chain are not respectively affected by the binding of another lectin to the second sugar chain, then $B_{21}/B_{22} = A_{21}/A_{22}$ is established and, as a result, the aforementioned relation (I) is established. In addition, when the second to $n^{th}$ lectins bind to the second sugar chain and if the first lectin is suppressed from binding to the second sugar chain, $B_{21}/B_{22} < A_{21}/A_{22}$ is established and, as a result, the aforementioned relation (I) is established.

The case where the second lectin is suppressed from binding to the first sugar chain if the first lectin binds to the first sugar chain structure of the first sugar chain may be exemplified by the case where the first and second sugar chains have a second sugar chain structure to which a second lectin can bind, and where the second lectin is suppressed from binding to the second sugar chain structure if the first lectin binds to the first sugar chain structure of the first sugar chain.

The case where the second lectin is suppressed from binding to the second sugar chain when the first lectin binds to the first sugar chain structure of the first sugar chain may be exemplified by the case where the first and second sugar chain structures in the first sugar chain are in the vicinity of each other, and the strength of binding of the first lectin to the first sugar chain structure is greater than the strength of binding of the second lectin to the second sugar chain structure. In that case, the binding of the first lectin to the first sugar chain structure competes with the binding of the second lectin to the second sugar chain structure; however, the strength of binding of the first lectin to the first sugar chain structure is greater, so the first lectin binds to the first sugar chain structure preferentially and the first lectin that has bound to the first sugar chain structure provides steric hindrance that suppresses the second lectin from binding to the second sugar chain structure. Note that the expression reading "the first and second sugar chain structures are in the vicinity of each other" may cover the case where part of the first sugar chain structure overlaps part of the second sugar chain structure.

In the method of the present invention, the method of measuring the amounts in which the first and second lectins are bound to the target sugar chain is not particularly limited. Take, for example, the case where labeling substances that can be detected as distinguished from each other are bound to the first and second lectins, respectively; in this case, the amounts in which the first and second lectins are bound to the target sugar chain can be measured on the basis of the amounts of the labeling substances.

In the method of the present invention, the target sugar chain to be mixed with the first to $n^{th}$ lectins is preferably fixed to a solid support. In that case, foreign substances adhering to the solid support (for example, sugar chains, etc. other than the target sugar chain, which are contained in a sample together with the target sugar chain), and lectins not bound to the target sugar chain can be easily removed by washing the solid support.

The material and form, etc. of the solid support to which the target sugar chain has been fixed are not particularly limited, but are preferably particles or magnetic particles. In that case, the binding of the first to $n^{th}$ lectins to the target sugar chain can proceed efficiently if the particles or magnetic particles to which the target sugar chain is fixed are dispersed into a liquid together with the first to $n^{th}$ lectins. Specifically, the handling of the particles is improved if they are magnetic particles and the magnetic particles dispersed in the liquid can be easily trapped by the action of a magnet, and the magnetic particles can be redispersed into the liquid by removing the action of the magnet.

In the method of the present invention, the target sugar chain may be a sugar chain bound to a protein or a lipid, etc. (specifically, a sugar chain contained in a glycoprotein or a glycolipid, etc.), or a sugar chain that is not bound to a protein or a lipid, etc., but a sugar chain contained in a glycoprotein is preferable. In that case, an antibody that can bind specifically to the protein part of a glycoprotein or fragment thereof is fixed to the solid support, and the sample is brought into contact with the solid support, whereby the target sugar chain in the sample can be fixed to the solid support. In addition, after the target sugar chain in the sample has been fixed to the solid support, the solid support is washed, whereupon the foreign substances contained in the sample can be separated from the target sugar chain.

According to the present invention, there is provided a method that can easily and efficiently detect a structural change in a target sugar chain that can be present as a first or second sugar chains depending on the structural change.

BRIEF DESCRIPTION OF THE DRAWINGS

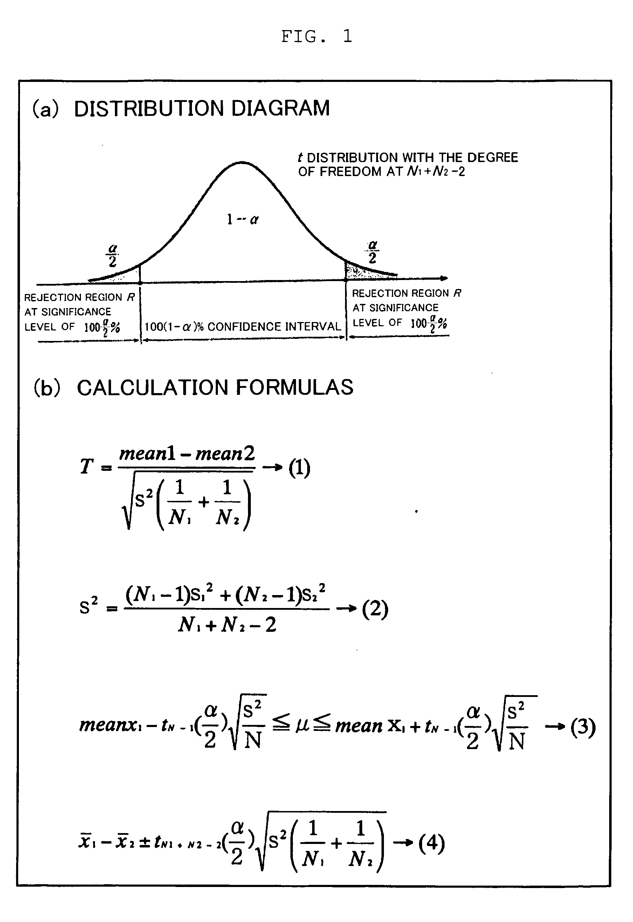

Fig. 1 is a diagram relating to T-test.

BEST MODE FOR CARRYING OUT THE INVENTION

The method according to the embodiment under consideration detects the presence or absence of a structural change in a target sugar chain T using as an index the ratio ($D_1/D_2$) between the amount ($D_1$) of lectin $L_1$ that binds to the target sugar chain T and the amount ($D_2$) of lectin $L_2$ that binds to the target sugar chain T when the target sugar chain T, which can be present as sugar chain $G_1$ or sugar chain $G_2$ depending on the structural change, is mixed with lectin $L_1$ and lectin $L_2$ that can bind to the sugar chains $G_1$ and $G_2$.

The target sugar chain T is a sugar chain contained in a glycoprotein. The glycoprotein containing the target sugar chain T is not particularly limited and may be exemplified by: physiologically active substances such as transferrin, blood group glycoprotein, human chorionic gonadotropin (hCG), thyroid stimulating hormone (TSH), and luteinizing hormone (LH); cancer related antigens such as prostate specific antigen (PSA), $\alpha$2-macroglobulin, carcinoembrionic antigen (CEA), and a-fetoprotein (AFP); and sugar chain antigens such as CA19-9, and CA125. Note that sugar chain antigens may not be bound to the cell surface or they may be bound to the cell surface (cell-surface sugar chain antigens).

The target sugar chain T is a sugar chain that can be present as sugar chain $G_1$ or $G_2$ depending on the sugar chain structure.

The structures of the sugar chains $G_1$ and $G_2$ differ in that the sugar chain $G_1$ has a sugar chain structure $S_1$ to which the lectin $L_1$ can bind, but the sugar chain $G_2$ does not have the sugar chain structure $S_1$, and the structures of the sugar chains $G_1$ and $G_2$ are substantially the same on all other points. Note that the expression reading "the structures of the sugar chains $G_1$ and $G_2$ are substantially the same" may also cover the case where those parts of sugar chains $G_1$ and $G_2$ to which the lectins $L_1$ and $L_2$ cannot bind have differences in sugar chain structure.

Preferably, the sugar chain $G_1$ is present as a single type of sugar chain with no difference in terms of sugar chain structure, but as long as the ratio ($B_{11}/B_{12}$) between the amount ($B_{11}$) of lectin $L_1$ binding to one molecule of sugar chain $G_1$ and the amount ($B_{12}$) of lectin $L_2$ binding to one molecule of sugar chain $G_1$ when sugar chain $G_1$, lectin $L_1$ and lectin $L_2$ have been mixed takes a fixed value or value range, the sugar chain $G_1$ may also be present as two or more types of sugar chain with a difference in terms of sugar chain structure.

Preferably, the sugar chain $G_2$ is present as a single type of sugar chain where there is no difference in terms of sugar chain structure, but as long as the ratio ($B_{21}/B_{22}$) between the amount ($B_{21}$) of lectin $L_1$ binding to one molecule of sugar chain $G_2$ and the amount ($B_{22}$) of lectin $L_2$ binding to one molecule of sugar chain $G_2$ when sugar chain $G_2$, lectin $L_1$ and lectin $L_2$ have been mixed takes a fixed value or value range, the sugar chain $G_2$ may also be present as two or more types of sugar chain where there is a difference in terms of sugar chain structure.

Structural changes in the target sugar chain T include the addition of a sugar chain structure $S_1$, the loss of a sugar chain structure $S_1$, a change from a sugar chain structure $S_1$ to a sugar chain structure $S_1$', and a change from a sugar chain structure $S_1$" to a sugar chain structure $S_1$. If the structural change is the addition of a sugar chain structure $S_1$, then the sugar chain prior to the structural change is the sugar chain $G_2$, and the sugar chain after the structural change is the sugar chain $G_1$; if the structural change is the loss of a sugar chain structure $S_1$, then the sugar chain prior to the structural change is the sugar chain $G_1$, and the sugar chain after the structural change is the sugar chain $G_2$; if the structural change is a change from a sugar chain structure $S_1$ to a sugar chain structure $S_1$', then the sugar chain prior to the structural change is the sugar chain $G_1$, and the sugar chain after the structural change is the sugar chain $G_2$; and if the structural change is a change from a sugar chain structure $S_1$" to a sugar chain structure $S_1$, then the sugar chain prior to the structural change is the sugar chain $G_2$, and the sugar chain after the structural change is the sugar chain $G_1$.

The causes of the structural changes in the target sugar chain T may include: diseases such as hepatocellular cancer, colon cancer, lung cancer, pancreatic cancer, thyroid cancer, myeloma, and chronic rheumatoid arthritis; processing by glycosidases such as sialidase, galactosidase, N-acetylglucosaminidase, mannosidase, and fucosidase; and differences of blood type. Even if structural changes are produced in the sugar chain contained in a glycoprotein, structural changes are not produced in the protein contained in the glycoprotein. Consequently, the protein bound to the sugar chain $G_1$ and the protein bound to the sugar chain $G_2$ have substantially the same structure.

If the target sugar chain T is a sugar chain contained in a-fetoprotein (AFP), the target sugar chain T has a-L-fucose residues, N-acetylglucosamine residues (bisecting N-acetylglucosamine) and the like added in association with the advance of hepatocellular cancer. In that case, the sugar chain structure added in association with the advance of hepatocellular cancer is the sugar chain structure $S_1$, the sugar chain prior to the addition of the sugar chain structure $S_1$ is the sugar chain $G_2$, and the sugar chain after the addition of the sugar chain structure $S_1$ is the sugar chain $G_1$.

The lectins $L_1$ and $L_2$ can bind to the sugar chains $G_1$ and $G_2$ in such a way as to satisfy the following relation (I):

Relation 2

$$\frac{B_{11}/B_{12}}{B_{21}/B_{22}} > \frac{A_{11}/A_{12}}{A_{21}/A_{22}} > 1$$

[wherein, $A_{11}$ represents the amount of lectin $L_1$ bound to one molecule of sugar chain $G_1$ when sugar chain $G_1$ and lectin $L_1$ are mixed in the absence of lectin $L_2$; $A_{12}$ represents the amount of lectin $L_2$ bound to one molecule of sugar chain $G_1$ when sugar chain $G_1$ and lectin $L_2$ are mixed in the absence of lectin $L_1$; $A_{21}$ represents the amount of lectin $L_1$ bound to one molecule of sugar chain $G_2$ when sugar chain $G_2$ and lectin $L_1$ are mixed in the absence of lectin $L_2$; $A_{22}$ represents the amount of lectin $L_2$ bound to one molecule of sugar chain $G_2$ when sugar chain $G_2$ and lectin $L_2$ are mixed in the absence of the lectin $L_1$; $B_{11}$ represents the amount of lectin $L_1$ bound to one molecule of sugar chain $G_1$ when sugar chain $G_1$ is mixed with lectin $L_1$ and lectin $L_2$; $B_{12}$ represents the amount of lectin $L_2$ bound to one

molecule of sugar chain $G_1$ when sugar chain $G_1$ is mixed with lectin $L_1$ and lectin $L_2$; $B_{21}$ represents the amount of lectin $L_1$ bound to one molecule of sugar chain $G_2$ when sugar chain $G_2$ is mixed with lectin $L_1$ and lectin $L_2$; and $B_{22}$ represents the amount of lectin $L_2$ bound to one molecule of sugar chain $G_2$ when sugar chain $G_2$ is mixed with lectin $L_1$ and lectin $L_2$. ]

Lectin $L_1$ can bind to a sugar chain structure $S_1$ of the sugar chain $G_1$, and it can also bind to sugar chain structures other than the sugar chain structure $S_1$. Preferably, the lectin $L_1$ shows a specific affinity to the sugar chain structure $S_1$. Here, the specific affinity of lectin $L_1$ to the sugar chain structure $S_1$, is not a strict one and even if the lectin $L_1$ shows a specific affinity to the sugar chain structure $S_1$, the lectin $L_1$ can also show affinity to sugar chain structures other than the sugar chain structure $S_1$.

The structures of the sugar chains $G_1$ and $G_2$ differ in that the sugar chain $G_1$ has a sugar chain structure $S_1$ to which the lectin $L_1$ can bind, but the sugar chain $G_2$ does not have the sugar chain structure $S_1$, and the structures of the sugar chains $G_1$ and $G_2$ are substantially the same on all other points. Therefore the amount ($A_{11}$) of lectin $L_1$ binding to one molecule of sugar chain $G_1$ is larger than the amount ($A_{21}$) of lectin $L_1$ biding to one molecule of sugar chain $G_2$, whereas the amount ($A_{12}$) of lectin $L_2$ binding to one molecule of sugar chain $G_1$ and the amount ($A_{21}$) of lectin $L_1$ binding to one molecule of sugar chain $G_2$ are substantially the same.

Consequently, $A_{11}/A_{12} > A_{21}/A_{22}$ is established.

Consequently, the aforementioned relation (I) is satisfied by selecting the lectins $L_1$ and $L_2$ such that $B_{11}/B_{12} > A_{11}/A_{12}$ and $A_{21}/A_{22} \geqq B_{21}/B_{22}$ are satisfied. In addition, the aforementioned relation (I) is satisfied by selecting the lectins $L_1$ and $L_2$ such that $B_{11}/B_{12} \geqq A_{11}/A_{12}$ and $A_{21}/A_{22} > B_{21}/B_{22}$ are satisfied.

When the sugar chain $G_1$, the lectin $L_1$, and lectin $L_2$ are mixed, $B_{11}/B_{12}$ can indicate a value or value range different than that of $A_{11}/A_{12}$ if the binding of either lectin $L_1$ or lectin $L_2$ to the sugar chain $G_1$ is affected by the other. Similarly, when the sugar chain $G_2$, the lectin $L_1$, and lectin $L_2$ are mixed, $B_{21}/B_{22}$ can indicate a value or value range different than that of $A_{21}/A_{22}$ if the binding of either lectin $L_1$ or lectin $L_2$ to the sugar chain $G_2$ is affected by the other. Consequently, lectins $L_1$ and $L_2$ can be selected such that $B_{11}/B_{12} > A_{11}/A_{12}$ and $A_{21}/A_{22} \geqq B_{21}/B_{22}$ are satisfied, and lectins $L_1$ and $L_2$ can also be selected such that $B_{11}/B_{12} \geqq A_{11}/A_{12}$ and $A_{21}/A_{22} > B_{21}/B_{22}$ are satisfied.

The case where the binding of either lectin $L_1$ or lectin $L_2$ to the sugar chain $G_1$ is affected by the other when the sugar chain $G_1$, is mixed with lectin $L_1$ and lectin $L_2$ may be exemplified by the case where lectin $L_2$ is suppressed from binding to the sugar chain $G_1$ when the lectin $L_1$ is bound to the sugar chain structure $S_1$ of the sugar chain $G_1$. In that case, $B_{11}$ and $A_{11}$ are substantially the same but $B_{12}$ is smaller than $A_{12}$ and therefore $B_{11}/B_{12} > A_{11}/A_{12}$ is established. Note that in the case where lectin $L_2$ can bind to a plurality of sugar chain structures in the sugar chain $G_1$, lectin $L_2$ may be suppressed from binding to one or more of these sugar chain structures.

Suppose $B_{11}/B_{12} > A_{11}/A_{12}$ is established. When the sugar chain $G_2$ is mixed with the lectin $L_1$ and lectin $L_2$ and if the bindings of the lectin $L_1$ and $L_2$ to the sugar chain $G_2$ are not respectively affected by the binding of another lectin to the sugar chain $G_2$, $B_{21}/B_{22} = A_{21}/A_{22}$ is established and, as a result, the aforementioned relation (I) is established. In addition, when the lectin $L_2$ binds to the sugar chain $G_2$ and if the lectin $L_1$ is suppressed from binding to the sugar chain $G_2$, $B_{21}/B_{22} < A_{21}/A_{22}$ is established and, as a result, the aforementioned equation (I) is established.

The case where the lectin $L_2$ is suppressed from binding to the sugar chain $G_1$ when the lectin $L_1$ binds to the sugar chain structure $S_1$ of the sugar chain $G_1$ may be exemplified by the case where sugar chains $G_1$ and $G_2$ have a sugar chain structure $S_2$ to which the lectin $L_2$ can bind, and where the lectin $L_2$ is suppressed from binding to the sugar chain structure $S_2$ when the lectin $L_1$ binds to the sugar chain structure $S_1$ of the sugar chain $G_1$.

The case where the lectin $L_2$ is suppressed from binding to the sugar chain structure $S_2$ when lectin $L_1$ binds to the sugar chain structure $S_1$ of the sugar chain $G_1$ may be exemplified by the case where sugar chain structures $S_1$ and $S_2$ in the sugar chain $G_1$ are in the vicinity of each other, and where the force of binding of the lectin $L_1$ to the sugar chain structure $S_1$ is greater than the force of binding of the lectin $L_2$ to the sugar chain structure $S_2$. In that case, the binding of the lectin $L_1$ to the sugar chain structure $S_1$ competes with the binding of the lectin $L_2$ to the sugar chain structure $S_2$; however, the strength of binding of the lectin $L_1$ to the sugar chain structure $S_1$ is greater, so the lectin $L_1$ binds to the sugar chain structure $S_1$ preferably and the lectin $L_1$ that has bound to the sugar chain structure $S_1$ provides steric hindrance that suppresses the lectin $L_2$ from binding to the sugar chain structure $S_2$. Note that the expression reading "the sugar chain structures $S_1$ and $S_2$ of the sugar chain $G_1$ are in the vicinity of each other" may cover the case where part of the sugar chain structure $S_1$ overlaps part of the sugar chain structure $S_2$.

As long as the lectins $L_1$ and $L_2$ can bind to sugar chains $G_1$ and $G_2$ in such a way as to satisfy the aforementioned relation (I), the lectins $L_1$ and $L_2$ are not particularly limited, and they can be suitably selected from known lectins such as, for example, Aleuria aurantia lectin (AAL), Agaricus bisporus lectin (ABA), Bacchinia purpura lectin (BPA), Bandeiraea Simplicifolia Agglutinin (BSA-II), concanavalin A (ConA), Dolichos biflorus lectin (DBA), Datura stramonium lectin (DSA), Erythrina cristagalli lectin (ECA), lentil lectin (LCA), Maackia amurensis agglutinin lectin (MAA (MAH)), Maackia amurensis lectin (MAM (MAL) ), peanut lectin (PNA), Pisum sativum lectin (PSA), pokeweed lectin (PWM), castor bean lectin (RCA120 or RCA60), soy bean lectin (SBA), Sambucus nigra lectin (SNA), Solanum tuberosum lectin (STA), Sambucus sieboldiana lectin (SSA), Ulex europaeus lectin (UEA-I), and wheat germ lectin (WGA).

The ratio $(D_1/D_2)$ between the amount $(D_1)$ of lectin $L_1$ binding to the target sugar chain T and the amount $(D_2)$ of lectin $L_2$ binding to the target sugar chain T when the target sugar chain T is mixed with lectin $L_1$ and lectin $L_2$ can be determined by the following steps (a) to (e).

Step (a): The target sugar chain T in a sample is fixed to a solid support.

As long as it contains the target sugar chain T, the sample is not particularly limited, and may be exemplified by samples derived in vivo such as blood serum, blood, blood plasma, urine, lymphocytes, hematocytes, and cells. More than one molecule of the target sugar chain T may be contained in the sample, and there are three possibilities: all of the molecules have undergone a structural change; some of the molecules have undergone a structural change; and none of the molecules have undergone a structural change.

The method of fixing the target sugar chain T to the solid support is not particularly limited, and can be performed following common methods. For example, an antibody that can bind specifically to the protein part of a glycoprotein containing the target sugar chain T or a fragment thereof is fixed to a solid support and then the sample is brought into contact with the solid support, whereby the target sugar chain T in the sample can be fixed to the solid support.

The material of the solid support is not particularly limited, and examples include: glass; silicon; ceramics; synthetic resins such as polystyrene resins such as polystyrene, acrylic resins (methacrylic resins) such as polymethyl methacrylate, polyamide resin, polyesters such as polyethylene terephthalate, and polycarbonate; polysaccharides such as agarose, dextran, and cellulose; and proteins such as gelatin, collagen, and casein.

The shape of the solid support is not particularly limited, and examples may include plate shapes, particle shapes, and the like, but particle shapes are preferable. If the solid support is particles, the target sugar chain T in the sample can be efficiently fixed to the particles by dispersing the particles in the sample. If the solid support is particles, they are preferably magnetic. If the solid support is magnetic particles, the handling of the particle is improved and the magnetic particles dispersed in the sample can be easily trapped by the action of a magnet, and the magnetic particles can be redispersed into the sample by removing the action of the magnet. These characteristics of magnetic particle are also useful when washing the solid support (refer to steps (b) and (d)).

The method of fixing the antibody or fragment thereof to the solid support is not particularly limited, and can be performed following common methods. In an exemplary method of fixing an antibody or fragment thereof to the solid support, a functional group that the solid support has on its surface is reacted with a functional group of the antibody or fragment thereof so that the antibody or fragment thereof is covalently bonded to the surface of the solid support. A carboxyl group, amino group and the like may be cited as functional groups that can form a covalent bond, and these functional groups can be introduced to the solid support and antibody or fragment thereof following common methods. If the solid support has carboxyl groups on its surface, the carboxyl groups are first activated by carbodiimides before they are reacted with the amino group of the antibody or fragment thereof, so that the antibody or fragment thereof can be fixed to the support though amide bonding. If the solid support has amino groups on its surface, the amino groups are first converted to a carboxyl group using a cyclic acid anhydride such as succinic anhydride before they are reacted with the amino group of the antibody or fragment thereof, so that the antibody or fragment thereof can be fixed to the support though amide bonding. Another method that can be employed is one involving a biotin-avidin system and in this method, a biotinylated antibody or fragment thereof is fixed to the surface of a solid support that has been coated with avidin or streptavidin.

Step (b): The solid support to which the target sugar chain T has been fixed is washed.

When the sample and the solid support are brought into contact, foreign substances contained in the sample (for example, sugar chains other than the target sugar chain T, and the like) might adhere to the solid support, but the foreign substances adhering to the solid support may be removed by washing the solid support.

Examples of the washing solution that can be used to wash the solid support include: solvents with comparatively high polarity such as water and alcohol; aqueous solutions containing salts such as sodium chloride, potassium chloride, magnesium chloride and the like; aqueous solutions containing alcohols; aqueous solutions containing proteins, nucleic acids and the like; and the aforementioned solvents or aqueous solutions containing a suitable pH buffering agent such as phosphate, tris and the like.

If the sample does not contain any foreign substances, then step (b) may be omitted.

Step (c): The target sugar chain T that has been fixed to the solid support is mixed with the lectins $L_1$ and $L_2$.

Labeling substances $LB_1$ and $LB_2$ that can be detected as discriminated from each other are preliminarily bound to the lectins $L_1$ and $L_2$, respectively. As long as they can be detected as discriminated from each other, the labeling substances $LB_1$ and $LB_2$ are not particularly limited, and may be exemplified by: fluorescent substances such as fluorescein, rhodamine, phycoerythrin and the like; chemiluminescent substances such as luminol, lucigenin, acridium ester and the like; bioluminescent substances such as luciferase, luciferrin and the like; enzymes such as alkali phosphatase, horseradish peroxidase and the like; radioisotopes such as $^{99m}$Tc, $^{131}$I, $^{125}$I, $^{14}$C, $^{3}$H and the like; semiconductor nanoparticles; oligonucleotides; magnetic bodies; and antibodies. Preferably, the amount in which the labeling substance binds to one molecule of lectin $L_1$ is substantially the same between molecules. The same applies to lectin $L_2$. If the amount in which the labeling substance binds to one molecule of a lectin is substantially the same between molecules, the amount of the lectin can be accurately determined from the amount of labeling substance.

The order of mixing is not particularly limited, and lectins $L_1$ and $L_2$ may first be mixed before the target sugar chain T is mixed; alternatively, one of the lectins $L_1$ and $L_2$ is first mixed with the target sugar chain and then the other lectin is mixed.

If the target sugar chain T is mixed with the lectins $L_1$ and $L_2$, the target sugar chain T is brought into contact with the lectin $L_1$, and the target sugar chain T is also brought into contact with the lectin $L_2$. If the target sugar chain T is present as the sugar chain $G_1$, the lectin $L_1$ binds to the sugar chain structure $S_1$ of the target sugar chain T and to sugar chain structures other than the sugar chain structure $S_1$, and the lectin $L_2$ binds to a specified sugar chain structure of the target sugar chain T. If the target sugar chain T is present as the sugar chain $G_2$, the lectin $L_1$ binds to a specified sugar chain structure of the target sugar chain T, and the lectin $L_2$ binds to a specified sugar chain structure of the target sugar chain T. The binding of lectins $L_1$ and $L_2$ to the target sugar chain T reaches equilibrium over time.

To mix the target sugar chain T with the lectins $L_1$ and lectin $L_2$, solvents may be used and examples include: an aqueous solution containing a salt such as sodium chloride, potassium chloride, magnesium chloride or the like; an aqueous solution containing alcohols; an aqueous solution containing proteins, nucleic acids or the like; and the aforementioned solvents or aqueous solutions containing a suitable pH buffering agent such as phosphate, tris or the like. The temperature is usually 4 to 60° C, preferably 25 to 37° C; the time is usually 1 to 120 minutes, preferably 5 to 60 minutes; and the pH is usually 2 to 10, preferably 5 to 8. The amounts in which lectins $L_1$ and $L_2$ are to be mixed shall be in excess of the amount in which target sugar chain T is mixed.

Step (d): The solid support is washed to remove the lectins $L_1$ and $L_2$ that have not been bound to the target sugar chain T.

Examples of washing solutions may include: solvents with comparatively high polarity such as water and alcohol; aqueous solutions containing salts such as sodium chloride, potassium chloride, magnesium chloride and the like; aqueous solutions containing alcohols; aqueous solutions containing proteins, nucleic acids and the like; and the aforementioned solvents or aqueous solutions containing a suitable pH buffering agent such as phosphate, tris and the like.

Step (e): The amounts of labeling substances $LB_1$ and $LB_2$ are measured, and the ratio ($D_1/D_2$) between the amount ($D_1$) of lectin $L_1$ binding to the target sugar chain T and the amount ($D_2$) of lectin $L_2$ binding to the target sugar chain T is determined based on the amounts of labeling substances $LB_1$ and $LB_2$.

The amounts of labeling substances $LB_1$ and $LB_2$ can be measured following common methods in accordance with the types of labeling substance $LB_1$ and $LB_2$.

If the lectin $L_1$ is preliminarily correlated to the amount of labeling substance $LB_1$, the amount ($D_1$) of binding of lectin $L_1$ to target sugar chain T can be determined, on the basis of the amount of labeling substance $LB_1$. Similarly, the amount ($D_2$) of binding of lectin $L_2$ to target sugar chain T can be determined on the basis of the amount of labeling substance $LB_2$.

Take case where the sugar chain $G_1$ is the sugar chain prior to a structural change and the sugar chain $G_2$ is the sugar chain after the structural change. If $B_{11}/B_{12} > D_1/D_2 \geqq B_{21}/B_{22}$, it can be concluded that the target sugar chain T is present only as the sugar chain $G_2$ or as a mixture of the sugar chains $G_1$ and $G_2$, in other words, the structural change has occurred in the target sugar chain T. Also assume the case where when the sugar chain $G_2$ is the sugar chain prior to a structural change and the sugar chain $G_1$ is the sugar chain after the structural changes. If $B_{11}/B_{12} \geqq D_1/D_2 > B_{21}/B_{22}$, it can be concluded that the target sugar chain T is present only as the sugar chain $G_1$ or as a mixture of the sugar chains $G_1$ and $G_2$, in other words, the structural change has occurred in the target sugar chain T.

In the embodiment under consideration, $D_1/D_2$ is used as the ratio between the amount ($D_1$) in which the lectin $L_1$ binds to the target sugar chain T and the amount ($D_2$) in which lectin $L_2$ ($D_2$) binds to the target sugar chain T but $D_2/D_1$ may also be used. In this alternative case, if $B_{12}/B_{11} < D_2/D_1 \leq B_{22}/B_{21}$, it can be concluded that the target sugar chain T is present only as the sugar chain $G_2$ or as a mixture of the sugar chains $G_1$ and $G_2$. In addition, if $B_{12}/B_{11} \leq D_2/D_1 < B_{22}/B_{21}$, it can be concluded that the target sugar chain T is present only as the sugar chain $G_1$ or as a mixture of the sugar chains $G_1$ and $G_2$.

In the embodiment under consideration, a sugar chain contained in a glycoprotein is used as the target sugar chain T, but a sugar chain contained in a glycolipid, or a sugar chain that is not bound to a protein, lipid or the like may also be used as the target sugar chain T. Exemplary glycolipids containing target sugar chain T may include galactocerebroside, glucocerebroside, globoside, lactosylceramide, trihexosylceramide, paragloboside, Forssman antigen glycolipid, fucosyl ganglioside, fucosylceramide, body type activated glycolipids, and the like.

Examples

Example 1

Preparation of anti-transferrin antibody bound magnetic beads, and structural analysis of transferrin sugar chains with mixed lections.

Transferrin (TF) (Serologicals Proteins, Inc.) was adsorbed specifically to anti-transferrin antibody (Inter-Cell Technologies, Inc.) bound magnetic beads. Thereafter, only when the sugar chain of transferrin was to be decomposed partially, sialidase was added to the magnetic bead suspension and allowed to react for removing the sialic acid present on the non-reducing terminal of the sugar chain. Next, the magnetic bead suspension was mixed with biotin labeled RCA lectin and FITC labeled SSA lectin and allowed to react, and by further allowing Cy5 labeled streptavidin to react, Cy5 was bound specifically to the RCA lectin. SSA lectin strongly recognizes the sialic acid containing Siaa2-6Gal/GalNAc structure present on the non-reducing terminal of the sugar chain, and RCA lectin recognizes the Galβ1-4GlcNAc structure in the sugar chain. For the sample treated with sialidase and the sample not treated with sialidase, the amounts of Cy5 and FITC on the magnetic bead groups were measured by fluorophotometry and their ratio R1 (amount of Cy5 / amount of FITC) was determined, and the ratio R2 of the R1 of the sialidase treated sample to the R1 of the sample not treated with sialidase (R1 of the sialidase treated sample / R1 of the sample not treated with sialidase) was determined.

In addition, for the sialidase treated sample and the sample not treated with sialidase, the magnetic bead suspension was mixed with biotin labeled RCA lectin in the absence of FITC labeled SSA lectin and the magnetic bead suspension was mixed with FITC labeled SSA lectin in the absence of biotin labeled RCA lectin, and the amounts of Cy5 and FITC on the magnetic bead groups were measured by fluorophotometry and their ratio R1 (amount of Cy5 / amount of FITC) as well as the ratio R2 of the R1 of the sialidase treated sample to the R1 of the sample not treated with sialidase (R1 of the sialidase treated sample / R1 of the sample not treated with sialidase) were determined.

Note that the system in which the magnetic bead suspension is mixed with biotin labeled RCA lectin and FITC labeled SSA lectin shall be called a "mixed system", and the system in which the magnetic bead suspension is mixed with one of biotin labeled RCA lectin or FITC labeled SSA lectin in the absence of the other shall be called a "non-mixed system".

Details of the experimental method are described below.

Preparation was conducted by the method described in the document attached to the magnetic beads, Dynabeads M-270 Carboxylic acid (manufactured by Dynal). First, 10 μL of Dynabeads suspension in undiluted form (30 μg of beads) was placed in a 1.5-mL Eppendorf tube, which was erected in a magnetic stand to separate the magnetic beads which were then washed. The magnetic beads were washed 3 times using 100 μL of MiliQ. After washing, the magnetic beads were suspended in 10 μL of 0.005M CMC [N-cyclohexyl-N'- (2-morpholinoethyl) carbodiimide methyl-p-toluensulfonate] dissolved in MiliQ, and the carboxyl group was activated by slow tilt rotation at 4° C for 10 minutes. After the supernatant was removed from the magnetic bead solution, the magnetic beads were suspended by adding 6 μL of 0.3M MES [2-(N-morpholino)ethane sulfonic acid] buffer (pH 4.8) and 4 μL of 0.005 M CMC, and the resulting suspension was subjected to slow tilt rotation at 4° C for 30 minutes. Next, after the supernatant was removed from the magnetic bead solution, the magnetic beads were washed twice in 100 μL of 0.1 M MES buffer (pH 4.8), suspended in 2 μL of the same buffer and 8 μL of anti-transferrin antibody (1 mg/mL), and the resulting suspension was subjected to slow tilt rotation at 4° C for 20 minutes. Then, 5 μL of 10-mg/mL BSA solution prepared with MiliQ was added to the magnetic bead solution, and after mixing, the mixture was subjected to slow tilt rotation at 4° C overnight. The following day, after washing the magnetic beads 3 times with 200 μL of PBS buffer, the magnetic beads were suspended by adding 1.1 μL of 10-mg/mL BSA solution prepared with MiliQ and 10 μL of an undiluted transferrin solution (1 mg/mL) in the order written, and the resulting suspension was subjected to slow tilt rotation at room temperature for 1 hour. Next, after removing the supernatant from the magnetic bead solution, the magnetic beads were washed 3 times with 200 μL of TBS buffer. Then, the magnetic beads were suspended by adding 97 μL of C-P buffer (50mM $Na_2PO_4$, 1M Citric acid) and 3 μL of sialidase (10 mU/μL) in the order written, and the resulting suspension was subjected to slow tilt rotation at 37° C for 3.5 hours. When no treatment with sialidase was performed, 3 μL of 1-mg/ml BSA solution prepared with MiliQ was added instead of sialidase. Next, after removing the supernatant from the magnetic bead solution, the magnetic beads were washed 3 times with 200 μL of TBS buffer. Next, after suspending the magnetic beads by adding 35 μL each of biotin labeled RCA lectin (0.3 mg/ml) and FITC labeled SSA lectin (0.3 mg/ml), as well as 3.85 μL of 10-mg/ml BSA solution prepared with MiliQ in the order written, the resulting suspension was subjected to slow tilt rotation at room temperature for 1 hour. In the non-mixed system, either biotin labeled RCA lectin or FITC labeled SSA lectin was added. Next, after removing the supernatant from the magnetic bead solution, the magnetic beads were washed 3 times with 200 μL of TBS buffer. Then, after suspending the magnetic beads by adding 20 μL of 2xBW buffer [10mM Tris-HCl (pH-7.5), 1mM EDTA, 2.0M NaCl] and 20 μL of Cy5 labeled streptavidin (0.3mg/ml) in the order written, the resulting suspension was subjected to slow tilt rotation at room temperature for 40 minutes. Next, after removing the supernatant from the magnetic bead solution, the magnetic beads were washed 3 times with 200 μL of 1×BW buffer. Then, the magnetic beads were suspended in 100 μL of TBS buffer and a 20-μL aliquot of the suspension was taken and diluted with 100 μL of TBS buffer; the dilution was placed in the wells of a 96-well black flat bottom plate, and the fluorescent intensities of the Cy5 and FITC were measured using a fluorescent plate reader.

The results of measurement on the mixed system are shown in Table 1, and those on the non-mixed system are shown in Table 2.

Table 1

| Sample No. | Not treated with Sialidase | | | Treated with Sialidase | | | |
|---|---|---|---|---|---|---|---|
| | Amount of FITC | Amount of Cy5 | Ratio R1 | Amount of FITC | Amount of Cy5 | Ratio R1 | Ratio R2 |
| 1 | 3.191 | 7.23 | 2.27 | 1.434 | 29.344 | 20.47 | 9.03 |
| 2 | 3.005 | 9.398 | 3.13 | 0.606 | 18.821 | 31.09 | 9.94 |
| 3 | 5.149 | 9.787 | 1.90 | 0.86 | 19.905 | 23.16 | 12.18 |
| 4 | 1.821 | 7.944 | 4.36 | 0.491 | 23.752 | 48.43 | 11.10 |
| 5 | 1.709 | 5.587 | 2.10 | 0.186 | 5.472 | 29.52 | 14.06 |
| 6 | 5.817 | 12.812 | 2.20 | 1.761 | 32.964 | 17.99 | 8.17 |
| 7 | 2.865 | 6.695 | 2.34 | 1.723 | 23.4 | 13.58 | 5.81 |
| 8 | 2.543 | 7.549 | 2.97 | 0.43 | 29.789 | 69.24 | 23.32 |
| 9 | 1.21 | 6.597 | 5.46 | 0.104 | 7.868 | 76.23 | 13.98 |
| 10 | 1.128 | 2.176 | 1.93 | 0.677 | 7.592 | 11.22 | 5.82 |
| 11 | 1.699 | 4.888 | 2.88 | 0.284 | 6.432 | 22.65 | 7.87 |
| 12 | 0.741 | 3.596 | 4.85 | 0.156 | 4.259 | 27.27 | 5.62 |
| 13 | 2.096 | 4.181 | 1.99 | 0.824 | 9.245 | 11.21 | 5.62 |
| 14 | 3.976 | 8.679 | 2.18 | 1.436 | 20.665 | 14.39 | 6.60 |
| 15 | 2.089 | 4.396 | 2.11 | 0.513 | 6.622 | 12.92 | 6.14 |
| 16 | 3.298 | 9.672 | 2.93 | 1.001 | 27.244 | 27.21 | 9.28 |
| Mean | 2.646 | 6.949 | 2.85 | 0.78 | 17.085 | 28.54 | 9.66 |
| Standard deviation | 1.41 | 2.76 | 1.10 | 0.55 | 10.11 | 19.74 | 4.46 |
| Relative standard deviation | 0.53 | 0.39 | 0.39 | 0.70 | 0.53 | 0.69 | 0.48 |

Table 2

| Sample No. | Not treated with Sialidase | | | Treated with Sialidase | | | |
|---|---|---|---|---|---|---|---|
| | Amount FITC | Amount of Cy5 | Ratio R1 | Amount of FITC | Amount of Cy5 | Ratio R1 | Ratio R2 |
| 1 | 5.899 | 14.242 | 2.41 | 1.973 | 26.306 | 13.3 | 5.5 |
| 2 | 4.94 | 7.997 | 1.62 | 1.627 | 19.331 | 11.9 | 7.3 |
| 3 | 4.939 | 6.961 | 1.41 | 0.846 | 15.324 | 18.1 | 12.8 |
| 4 | 6.827 | 7.734 | 1.13 | 1.772 | 21.197 | 11.9 | 10.5 |
| 5 | 3.751 | 3.965 | 1.06 | 1.968 | 6.122 | 3.11 | 2.93 |
| 6 | 3.3 | 2.646 | 0.8 | 1.733 | 3.679 | 2.12 | 2.62 |
| 7 | 3.16 | 3.141 | 1 | 0.736 | 5.081 | 6.9 | 6.9 |
| 8 | 2.606 | 2.476 | 0.95 | 0.709 | 3.549 | 5 | 5.26 |
| 9 | 3.416 | 3.173 | 0.93 | 1.761 | 4.964 | 2.82 | 3 |

(continued)

| Sample No. | Not treated with Sialidase | | | Treated with Sialidase | | | |
| --- | --- | --- | --- | --- | --- | --- | --- |
| | Amount FITC | Amount of Cy5 | Ratio R1 | Amount of FITC | Amount of Cy5 | Ratio R1 | Ratio R2 |
| 10 | 3.289 | 1.799 | 0.54 | 1.303 | 2.089 | 1.62 | 2.95 |
| 11 | 4.018 | 11.256 | 2.8 | 0.729 | 19.902 | 27.3 | 9.7 |
| Mean | 4.195 | 5.945 | 1.33 | 1.378 | 11.595 | 9.46 | 6.31 |
| Standard deviation | 1.304 | 4.063 | 0.7 | 0.526 | 8.863 | 8.04 | 3.49 |
| Relative standard deviation | 0.31 | 0.68 | 0.52 | 0.38 | 0.76 | 0.85 | 0.55 |

As shown in Tables 1 and 2, the ratio R2 of the mixed system (mean 9.66) was larger than the ratio R2 of the non-mixed system (mean 6.31).

The reason would be as follows.

The SSA binding site of the transferring sugar chain partially overlaps its RCA binding site, so in the sample of the mixed system which was not treated with sialidase, the binding of SSA to the transferring sugar chain competes with the binding of RCA and the binding of SSA occurs preferentially and, as a result, the binding of RCA is suppressed. On the other hand, in the sialidase treated sample of the mixed system, the removal of sialic acid suppresses SSA from binding to the transferring sugar chain and, as a result, RCA is not suppressed from binding to the transferring sugar chain.

In the non-mixed system, both the sialidase treated sample and the sample not treated with sialidase are such that the binding of the RCA to the transferrin sugar chain is not suppressed because the binding of RCA to the transferrin sugar chain does not compete with the binding of SSA.

Consequently, the amount of RCA binding to one molecule of the transferring sugar chain is smaller in the sample of the mixed system not treated with sialidase than in the sample of the non-mixed system not treated with sialidase whereas said amount of RCA binding is substantially the same between the sialidase treated sample of the mixed system and the sialidase treated sample of the non-mixed system. Note that the amount of SSA binding to one molecule of the transferring sugar chain appears to be substantially the same between the sample of the mixed system not treated with sialidase and the sample of the non-mixed system not treated with sialidase and between the sialidase treated sample of the mixed system and the sialidase treated sample of the non-mixed system.

For that reason, the ratio R1 of the mixed system indicates a value or value range that is more reflective of the presence or absence of sialic acid, and the difference between the ratio R1 (2.85) of the sample of the mixed system not treated with sialidase and the ratio R1 (28.54) of the sialidase treated sample of the mixed system is larger than the difference between the ratio R1 (1.33) of the sample of the non-mixed system not treated with sialidase and the ratio R1 (9.46) of the sialidase treated sample of the non-mixed system, and as a result, the ratio R2 (mean 9.66) of the mixed system is larger than the ratio R2 (mean 6.31) of the non-mixed system (Mean value of the ratio R2 of the mixed system / mean value of the ratio R2 of the non-mixed system = 1.53). This tendency was also observed even when the combination of RCA and SSA was replaced by a combination of concanavalin A (ConA) and wheat germ lectin (WGA) in order to detect changes in the transferrin sugar chain structure that occurred in a treatment with β-mannosidase. Specifically, transferrin (TF) (Serologicals Proteins, Inc.) was adsorbed specifically to anti-transferrin antibody (Inter-Cell Technologies, Inc.) bound magnetic beads. Thereafter, only when the transferrin sugar chain was to be decomposed partially, β-mannosidase was added to the magnetic bead suspension and allowed to react. Next, the magnetic bead suspension was mixed with biotin labeled ConA and FITC labeled WGA and allowed to react (reaction solvent: TBS+0.5M NaCl, the concentration of each lectin: 0.3mg/mL), and by further allowing Cy5 labeled streptavidin to react, Cy5 was bound specifically to the ConA. Because the mannose was cut from the transferrin sugar chain by β-mannosidase treatment, the binding of WGA to the transferrin sugar chain remained nearly the same but the binding of ConA to the transferrin sugar chain became weak. For the β-mannosidase treated sample and the sample not treated with β-mannosidase, the amounts of Cy5 and FITC on the magnetic bead groups were measured by fluorophotometry and their ratio R1 (amount of Cy5 / amount of FITC) was determined, and the ratio R2 of the R1 of the β-mannosidase treated sample to the R1 of the sample not treated with β-mannosidase (R1 of the β-mannosidase treated sample / R1 of the sample not treated with β-mannosidase) was determined (Ratio R2 of the mixed system). In addition, for the β-mannosidase treated sample and the sample not treated with β-mannosidase, the magnetic bead suspension was mixed with biotin labeled ConA in the absence of FITC labeled WGA and the magnetic bead suspension was mixed with FITC labeled WGA in the absence of biotin labeled ConA, and the amounts of Cy5 and FITC on the magnetic bead groups were measured by fluorophotometry and their ratio R1 (amount of Cy5 / amount of FITC) was determined, and the ratio R2 of the R1 of the β-mannosidase treated sample to the R1 of the sample not treated with β-mannosidase (R1 of the β-mannosidase treated sample / R1 of the sample not treated with β-mannosidase) was determined (Ratio R2 of the non-mixed system). The results were: mean value (1.24) of the ratio R2 of the mixed system / mean value (0.77) of the ratio R2 of the non-mixed system = 1.61.

Consequently, if the ratio R1 of the mixed system is taken as an index, the structural changes of the transferrin sugar chain due to sialidase treatment or β-mannosidase treatment can be detected with better precision than when the ratio R1 of the non-mixed system is taken as an index. In addition, the fluctuations of the measured values in the mixed system were smaller than the fluctuations of the measured values in the non-mixed system, and if the ratio R1 of the mixed system is taken as an index, the structural changes of the transferrin sugar chain due to sialidase treatment can be detected with better precision than when the ratio R1 of the non-mixed system is taken as an index.

These facts are verified by the following statistical processing of reliability.

Suppose a test that evaluates whether a given sample is treated with sialidase or not. Then, the results of measurement on the mixed system and the results of measurement on the non-mixed system were assumed as two population mean values, and the difference between the two population mean values was tested (T-test).

Hypothesis H0: $\mu1=\mu2$

The population variance was unknown but $\sigma1=\sigma2$ was assumed and the test statistical quantity T in that case was calculated based on formulas (1) and (2) shown in Fig. 1.

Here, the meanings of the variables are as follows:

$\mu$ : Mean value of normal population
$\sigma$: Variance of normal population
m: Mean of specimen
s: Standard deviation
N: Degrees of freedom or size of specimen (sample)
t: t distribution
T: Value of T obtained from T-test
Mean: Mean value

In the mixed system, the T value is 5.1976 when the significance level is 5%, and the percent point of $t(N1+N_2-2) a/2$ is 2.04227. On the other hand, in the non-mixed system, the T value is 3.3411, and the percent point of $t(N1+N_2-2) a/2$ is 2.085962. Both the T values of the mixed and non-mixed systems are equal to or greater than $t(N1+N_2-2) a/2$, and thus $T \in R$ (rejection region) (refer to Fig. 1). When the significance level is 5%, hypothesis H0 is rejected. In other words, the two population means are not the same in the mixed and non-mixed systems.

The mean value of the normal population is calculated based on formula (3) in Fig. 1. For the sample not treated with sialidase, the confidence interval of the mixed system is $2.85-0.58 \leq \mu \leq 2.85+0.58$, and the confidence interval of the non-mixed system is $1.33-0.47 \leq \mu \leq 1.33+0.47$. For the sialidase treated sample, the confidence interval of the mixed system is $28.54-10.53 \leq \mu \leq 28.54+10.53$, and the confidence interval of the non-mixed system is $9.46-5.40 \leq \mu \leq 9.46+5.40$.

At a significance level of 5%, the confidence interval 100 (1-a) % for the difference of the two population means is calculated based on formula (4) in Fig. 1; the mixed system has a value of $25.69 \pm 10.09$, and the non-mixed system has a value of $8.13 \pm 5.07$.

If the non-mixed system is assumed to have the same error range of 10.09 (95% reliable) as the mixed system, the percent point of $t (N_1+N_2-2) a/2$ is calculated to be 4.1483 and the confidence interval is estimated to be 99.9%. Therefore, the mixed system is believed to be more reliable than the non-mixed system.

Example 2

The sugar chain structure of the tumor marker thyroglobulin was identified by repeating the method described in Example 1, except that transferrin and anti-transferrin antibody were changed to thyroglobulin (Biogenesis) and anti-thyroglobulin antibody (SIGMA) respectively. The results of measurement on the mixed system are shown in Table 3, and the results of measurement on the non-mixed system are shown in Table 4.

Table 3

| Sample No. | Not treated with Sialidase | | | Treated with Sialidase | | | |
|---|---|---|---|---|---|---|---|
| | Amount of FITC | Amount of Cy5 | Ratio R1 | Amount of FITC | Amount of Cy5 | Ratio R1 | Ratio R2 |
| 1 | 4.358 | 3.801 | 0.87 | 2.042 | 24.954 | 12.22 | 14 |
| 2 | 4.298 | 4.06 | 0.94 | 2.235 | 37.45 | 16.76 | 17.74 |
| Mean | 4.33 | 3.93 | 0.91 | 2.14 | 31.20 | 14.49 | 15.87 |

Table 4

| Sample No. | Not treated with Sialidase | | | Treated with Sialidase | | | |
|---|---|---|---|---|---|---|---|
| | Amount of FITC | Amount of Cy5 | Ratio R1 | Amount of FITC | Amount of Cy5 | Ratio R1 | Ratio R2 |
| 1 | 3.63 | 1.99 | 0.55 | 3.465 | 20.542 | 5.9 | 10.7 |

As shown in Tables 3 and 4, the difference for the mixed system between the ratio R1 of the sample not treated with sialidase and the ratio R1 of the sialidase treated sample was greater than the difference for the non-mixed system between the ratio R1 of the sample not treated with sialidase and the ratio R1 of the sialidase treated sample and, as a result, the ratio R2 of the mixed system was greater than the ratio R2 of the non-mixed system.

Consequently, if the ratio R1 of the mixed system is taken as an index, the structural changes of the thyroglobulin sugar chain that result from sialidase treatment can be detected more precisely than when the ratio R1 of the non-mixed system is taken as an index.

## Claims

1.    A method for detecting the presence or absence of a structural change in a target sugar chain, in which the target sugar chain which can be present as a first or second sugar chain depending on the structural change is mixed with a first to an nth lectin (n is an integer of 2 or more) that can bind to the first and second sugar chains, and the ratio between the amount of the first lectin binding to the sugar chain and the amount of the second lectin binding to the sugar chain is used as an index,
the first to the nth lectin satisfying the following relation (I):

Relation 1

$$\frac{B_{11}/B_{12}}{B_{21}/B_{22}} > \frac{A_{11}/A_{12}}{A_{21}/A_{22}} > 1$$

[wherein, $A_{11}$ represents the amount of the first lectin binding to one molecule of the first sugar chain when the first sugar chain and the first lectin are mixed in the absence of another lectin; $A_{12}$ represents the amount of the second lectin binding to one molecule of the first sugar chain when the first sugar chain and the second lectin are mixed in the absence of another lectin; $A_{21}$ represents the amount of the first lectin binding to one molecule of the second sugar chain when the second sugar chain and the first lectin are mixed in the absence of another lectin; $A_{22}$ represents the amount of the second lectin binding to one molecule of the second sugar chain when the second sugar chain and the second lectin are mixed in the absence of another lectin; $B_{11}$ represents the amount of the first lectin binding to one molecule of the first sugar chain when the first sugar chain is mixed with the first to the $n^{th}$ lectin; $B_{12}$ represents the amount of the second lectin binding to one molecule of the first sugar chain when the first sugar chain is mixed with the first to the $n^{th}$ lectin; $B_{21}$ represents the amount of the first lectin binding to one molecule of the second sugar chain when the second sugar chain is mixed with the first to the $n^{th}$ lectin; and $B_{22}$ represents the amount of the second lectin binding to one molecule of the second sugar chain when the second sugar chain is mixed with the first to the $n^{th}$ lectin.]

2. The method according to Claim 1, wherein the structures of the two sugar chains differ in that the first sugar chain has a first sugar chain structure to which the first lectin can bind, but the second sugar chain does not have the first sugar chain structure, and the two sugar chain structures are substantially the same on all other points.

3. The method according to Claim 2, wherein the second lectin is suppressed from binding to the first sugar chain if the first lectin binds to the first sugar chain structure of the first sugar chain.

4. The method according to Claim 3, wherein the first and second sugar chains have a second sugar chain structure to which the second lectin can bind, and
the second lectin is suppressed from binding to the second sugar chain structure if the first lectin binds to the first sugar chain structure of the first sugar chain.

5. The method according to any one of Claims 1 to 4, wherein labeling substances that can detected as discriminated from each other are bound to the first and second lectins, respectively, and the amounts in which the first and second lectins are bound to the target sugar chain are measured on the basis of the amounts of the labeling substances.

6. The method according to any one of Claims 1 to 5, wherein the target sugar chain to be mixed with the first to $n^{th}$ lectins is fixed on a solid support.

7. The method according to Claim 6, wherein the solid support is particles or magnetic particles.

8. The method according to any one of Claims 1 to 7, wherein the target sugar chain is a sugar chain contained in a glycoprotein.

FIG. 1

## (a) DISTRIBUTION DIAGRAM

$t$ DISTRIBUTION WITH THE DEGREE OF FREEDOM AT $N_1 + N_2 - 2$

$1 - \alpha$

$\frac{\alpha}{2}$

$\frac{\alpha}{2}$

REJECTION REGION $R$ AT SIGNIFICANCE LEVEL OF $100\frac{\alpha}{2}\%$ | $100(1-\alpha)\%$ CONFIDENCE INTERVAL | REJECTION REGION $R$ AT SIGNIFICANCE LEVEL OF $100\frac{\alpha}{2}\%$

## (b) CALCULATION FORMULAS

$$T = \frac{mean1 - mean2}{\sqrt{S^2\left(\frac{1}{N_1} + \frac{1}{N_2}\right)}} \rightarrow (1)$$

$$S^2 = \frac{(N_1 - 1)S_1^2 + (N_2 - 1)S_2^2}{N_1 + N_2 - 2} \rightarrow (2)$$

$$meanx_1 - t_{N-1}(\frac{\alpha}{2})\sqrt{\frac{S^2}{N}} \leqq \mu \leqq mean\, x_1 + t_{N-1}(\frac{\alpha}{2})\sqrt{\frac{S^2}{N}} \rightarrow (3)$$

$$\bar{x}_1 - \bar{x}_2 \pm t_{N1+N2-2}(\frac{\alpha}{2})\sqrt{S^2\left(\frac{1}{N_1} + \frac{1}{N_2}\right)} \rightarrow (4)$$

## INTERNATIONAL SEARCH REPORT

| International application No. |
|---|
| PCT/JP2005/015686 |

A.   CLASSIFICATION OF SUBJECT MATTER
Int.Cl⁷  G01N33/543, G01N33/553, G01N33/566

According to International Patent Classification (IPC) or to both national classification and IPC

B.   FIELDS SEARCHED

Minimum documentation searched (classification system followed by classification symbols)
Int.Cl⁷  G01N33/543, G01N33/553, G01N33/566

Documentation searched other than minimum documentation to the extent that such documents are included in the fields searched
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CA

C.   DOCUMENTS CONSIDERED TO BE RELEVANT

| Category* | Citation of document, with indication, where appropriate, of the relevant passages | Relevant to claim No. |
|---|---|---|
| A | MATSUMOTO, K. et al., "Antibody-lectin sandwich enzyme immunoassay for determination of altered asparagines-linked sugar chains in serum transferrin of patients with hepatoma", Jpn.J.Clin.Chem., 1994, Vol.23, pages 292 to 298 | 1-8 |
| A | OKAZAKI, I. et al., "Determination of the interactions between lectins and glycoproteins by surface plamon resonance", J.Mol.Recognition, 1995, Vol.8, pages 95 to 99 | 1-8 |
| A | JP 07-325083 A  (Nakarai Tesque Kabushiki Kaisha), 12 December, 1995 (12.12.95), (Family: none) | 1-8 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 September, 2005 (29.09.05) | 18 October, 2005 (18.10.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

| International application No. |
| --- |
| PCT/JP2005/015686 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 09-159669 A  (Nakano Vinegar Co., Ltd.), 20 June, 1997 (20.06.97), (Family: none) | 1-8 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2002066634 A
- JP 3070418 B